# EUROPEAN PATENT APPLICATION

(11) **EP 4 410 331 A1**
(43) Date of publication of application: **07.08.2024**
(21) Application number: 22876369.4
(22) Date of filing: 28.09.2022
(51) Int. Cl.: A61M 5/315

(54) **SYRINGE**

(30) Priority: 29.09.2021 JP 2021159771
(71) Applicant: TSK Laboratory, Japan, Tochigi 328-0002 (JP)
(72) Inventor: FUJISAWA, Toru, Tochigi-shi, Tochigi 328-0002 (JP)
(74) Representative: Serjeants LLP
(86) International application number: PCT/JP2022/036244
(87) International publication number: WO 2023/054503

(57) **Abstract**

In order to provide a metered dosing component which enables metering other than visually, there is provided a metered dosing component including; a plunger 107having a protruding portion on an outer surface thereof, a flange cover 109 to be placed over a flange portion 114 of the syringe 111, having a hole 116 through which the plunger passes, and a recess 125provided in the inner surface of the hole 116 through which the protruding portion passes, wherein the protruding portion has a first protruding portion 115extending axially to a front end side of the plunger, a second protruding portion 117 extending axially to a rear end side of the plunger and located at a different position from the first protruding portion in the circumferential direction, and a continuous protruding portion 119 connecting a rear end of the first protruding portion and a front end of the second protruding portion in a continuous manner, when the plunger is pulled backward from a position where the second protruding portion is located in the recess, the second protruding portion, the continuous protruding portion, and the first protruding portion pass through the recess in that order, and when the rear end of the first protruding portion passes through the recess, the plunger rotates with respect to the flange cover.

## Description

### Technical Field

This invention relates to a metered dosing component and an injection device.

### Background

There are many treatments in which a predetermined amount of drug solution is dosed, such as vaccine dosing and botulinum toxin dosing. Syringes are generally provided with drug solution with an amount which does not necessarily correspond to the amount required for the treatment such as 1mL, 2mL and 10mL. In treatments using such syringes, the amount of drug solution printed on the syringe is visually confirmed before dosing, which may result in dosing the wrong amount of drug solution. For drug solution which needs to be metered and dosed, if wrong amount of drug solution is dosed, it will not only have an effect on the affected area but also have a negative effect on the human body, such as a case of botulinum toxin.

. Therefore, in order to facilitate the delivery of desired amount of drug solution selectively only when necessary, Patent document 1 discloses a syringe comprising an outer cylinder that can be filled with liquid, a pusher that can move along the longitudinal direction of the outer cylinder within the outer cylinder and is formed with a plurality of concave and convex portions aligned in the longitudinal direction, a projection that is in contact with at least the convex portions sequentially as the pusher moves, and a pusher that is in contact with the convex portions sequentially as the pusher moves, and a rotation restricting portion of the pusher in contact with the pusher, wherein a ring shaped attachment that can be attached to the outer cylinder is provided.

. However, since such a syringe adjusts an amount of drug solution while dosing, there was a possibility of dosing wrong amount of drug solution.

. In order to discharge an exact amount of drug solution with a simple operation, Patent document 2 discloses a metered dosing syringe comprising an outer cylinder, a gasket that can slide within the outer cylinder, a pusher that moves and operates the gasket, and a stopper that is slidably installed along the longitudinal direction of the pusher, wherein the stopper is fixed with the pusher by its engaging portion engaging with a rack of the pusher, and when a pushbutton is pressed, the stopper is released from the fixed state and the position of the stopper on the pusher can be adjusted, and when the pushbutton is pressed toward the tip, the tip surface of the stopper contacts the base of the outer cylinder, thereby regulating the depth of insertion of the pushbutton into the outer cylinder, and thereby a predetermined amount of drug is ejected from the reduced diameter portion.

However, such a metered dosing syringe has a large number of additional parts, and its structure is complicated and cannot be manufactured inexpensively. In addition, there are many operating procedures for metered dosing, and it takes a long time to dose drug solution.

### Prior Art

### Patent Document

Patent Document 1: WO2013/161434A
Patent Document 2: JP 2003-199828A

### Summary

### Problem to be solved

It is an object of the present invention to provide a metered dosing component and an injection device using the same, which enable metered dosing other than visual checking and eliminate incorrect dosing. It is also an object of the present invention to provide a metered dosing component and a syringe using the same to eliminate the waste of drug solution more than required for air release by confirming a metered dosing start position of the plunger. It is further an object of the present invention to provide a metered dosing component and an injection device using the same at a low cost by changing the plunger of a general-purpose syringe and adding a flange cover. Means to solve Problem

. The metered dosing component of the present invention is
a metered dosing component including;
   a plunger having a protruding portion on an outer surface thereof,
   a flange cover to be placed over a flange portion of a syringe, having a hole through which the plunger passes, and a recess provided in an inner surface of the hole through which the protruding portion passes, wherein
the protruding portion has a first protruding portion extending axially to a front end side of the plunger, a second protruding portion extending axially to a rear end side of the plunger and located at a different position from the first protruding portion in the circumferential direction, and a continuous protruding portion connecting a rear end of the first protruding portion and a front end of the second protruding portion in a continuous manner,
when the flange cover is attached to the flange portion of the syringe and the plunger is inserted into the syringe, and the plunger is pulled backward from a position where the second protruding portion is located in the recess, the second protruding portion, the continuous protruding portion, and the first protruding portion pass through the recess in that order, and when the rear end of the first protruding portion passes through the recess, the plunger rotates with respect to the flange cover.

. According to the above configuration, the plunger rotates at the air release position of the plunger, thereby making it easy to determine an amount of drug solution required for air release.

### Effects of the Invention

The present invention can provide a metered dosing component and an injection device using the same, which enable metered dosing other than visual checking and eliminate incorrect dosing. It can also provide a metered dosing component and an injection device using the same, to eliminate the waste of drug solution more than required for air release by confirming a metered dosing start position of the plunger. It can further provide a metered dosing component and an injection device using the same at a low cost by changing the plunger of a general-purpose syringe and adding a flange cover.

### Brief Description of Drawings

Fig. 1: Schematic perspective view of an injection device according to an embodiment of the present invention
Fig. 2: Schematic exposed perspective view of the injection device according to the embodiment of the present invention
Fig. 3: A plan view of a plunger according to a first embodiment of the present invention.
Fig. 4: A cross-sectional view of the respective positions of the plunger in the flange cover according to the embodiment of the present invention
Fig. 5: A figure to show the operation of the plunger according to the first embodiment of the present invention.
Fig. 6: A plan view of a plunger according to a second embodiment of the present invention
Fig. 7: A cross-sectional view of the injection device pushing the plunger according to the embodiment of the present invention

### Detail Description

Various embodiments for implementing the present invention will be described below with reference to drawings. A metered dosing component and an injection device described below are intended to embody the technical concept of the present disclosure, and unless otherwise specifically stated, the disclosure is not limited to the following. For ease of explanation or understanding of the main points, similar components in different drawings are shown with the same reference numeral for convenience. In particular, similar effects of similar configurations shall not be mentioned sequentially for each embodiment or example. The size and positional relationship of the parts and materials shown in each drawing may be exaggerated for the sake of clarity of explanation.

.

### (Injection Device according to an embodiment of the present invention)

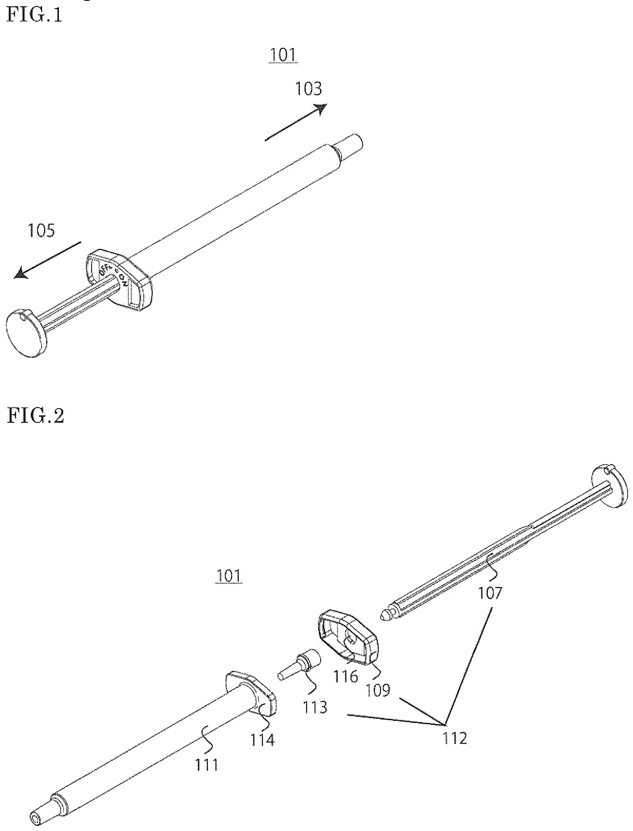

Figure 1 is a schematic perspective view of an injection device according to an embodiment of the present invention. Fig. 2: is a schematic exposed perspective view of the injection device according to the embodiment of the present invention. The injection device according to the embodiment of the present invention will be described with referring to Figs. 1 and 2.

.

In Figure 1, an injection device 101 of this embodiment is shown in a state before a needle hub with a needle is attached thereto. The arrow direction 103 with the needle in the injection device is the front end, front end side, front end direction, front end portion, front, etc., and the arrow direction 105 with the plunger inserted into the syringe is the back end, back end side, back end direction, back end portion, back, etc.

As shown in Figure 2, the injection device 101 has a syringe 111 in which drug solution is stored, a plunger 107 which is attached to the syringe 111 and pushes drug solution in the syringe 111, and a flange cover 109 which covers a flange portion 114 of the syringe 111. The flange cover 109 has a hole 116 through which the plunger 107 passes to secure the plunger 107. A gasket 113 is attached to the front end of the plunger 107. The gasket 113 attached to the front end allows the plunger 107 to perform the function of a piston that pushes drug solution in the syringe 111 to the front side without leakage

As shown in Figure 2, since the gasket 113 does not pass through the hole 116 in the flange cover 109, the flange cover 109 is assembled so that it is sandwiched between the plunger 107 and the gasket 113. In this embodiment, the injection device 101 is formed by attaching the metered dosing component 112, which is configured by the plunger 107 with the gasket 113 and flange cover 109, to a general-purpose syringe 111.

. The syringe 111 is preferably made of resin such as polypropylene. The plunger 107 is preferably made of resin such as polypropylene and ABS. The flange cover 109 is preferably made of resin such as polypropylene and ABS. The gasket is preferably entirely made of resin such as silicone and elastomer.

.

### (Metered dosing component according to First embodiment)

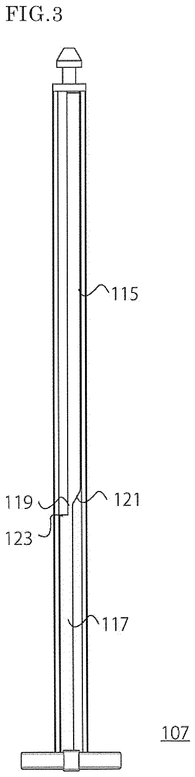

Fig. 3 is a plan view of a plunger according to a first embodiment of the present invention. A metered dosing component according to the first embodiment of the present invention will be described with referring to Fig.3.

. A metered dosing component 112 in this embodiment has a plunger 107, a flange cover 109, and a gasket 113. As shown in Fig. 3, the plunger 107 has protruding portions 115, 117, and 119 on an outer surface thereof. The protruding portions has a first protruding portion 115 extending axially to the front end side of the plunger 107, a second protruding portion 117 extending axially to the rear end side of the plunger 107 and located at a different position from the first protruding portion 115 in the circumferential direction, and a continuous protruding portion 119 connecting the rear end of the first protruding portion 115 and the front end of the second protruding portion 117 in a continuous manner.

. The rear end of the first protruding portion 115 has a surface 121 inclined with respect to the axial direction. The inclined surface 121 is connected to the continuous protruding portion 119 like a steep ridgeline in the axial and radial direction. The continuous protruding portion 119 is circumferentially shorter than the first protruding portion 115 and the second protruding portion 117 and is formed to connect the first protruding portion 115 and the second protruding portion 117 at a circumferentially overlapping portion. The continuous protruding portion 119 preferably extends parallel to the axial direction.

. The front end of the second projection 117 has a surface 123 that is perpendicular to the axial direction. This surface contacts the flange cover 109 and serves as a stopper. Therefore, it is preferably precipitous in the axial and radial direction.

### (Movement of Metered dosing component during drug drawing and drug dosing)

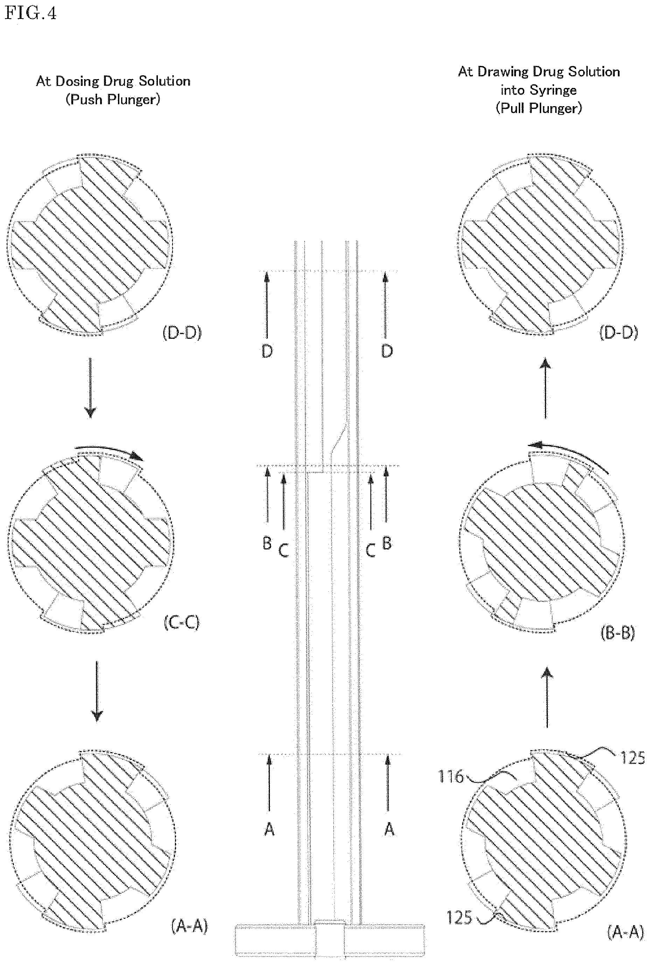

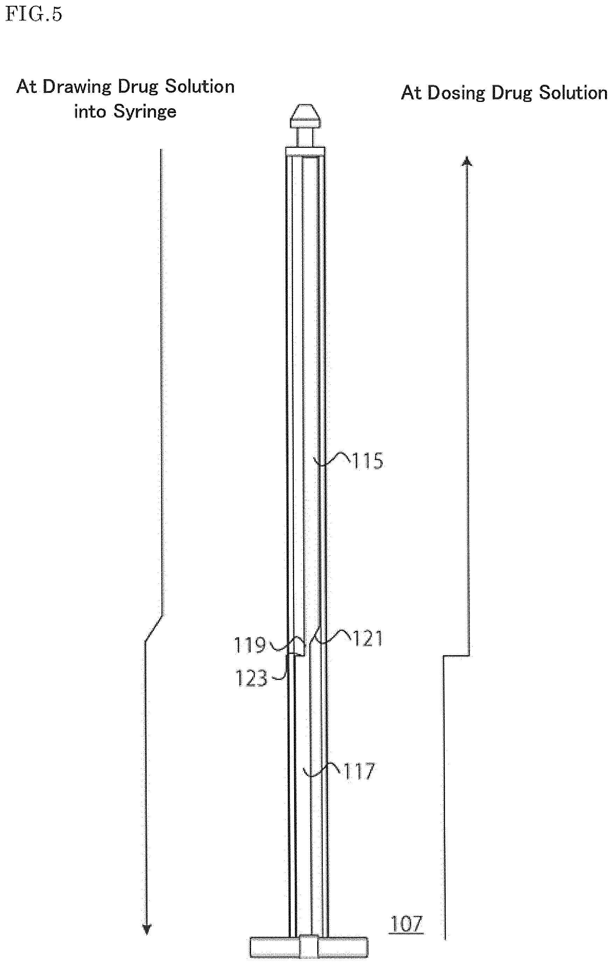

Fig. 4: is a cross-sectional view of the respective positions of the plunger in the flange cover according to the embodiment of the present invention. Fig. 5 is a figure to show the operation of the plunger according to the first embodiment of the present invention. With referring to Figs. 4 and 5, movement of the metered dosing component 112 when the plunger is withdrawn from the syringe (during drug drawing) and when the plunger is pushed in (during drug dosing) will be described

. First, the plunger 107 is inserted through the flange cover 109 and the gasket 113 is attached to the plunger 107, thereby forming the metered dosing component 112. Then, the flange portion 114 of the syringe 111 is covered with the flange cover 109. As shown in Figure 4 with dotted lines, the flange cover 109 has a hole 116 through which the plunger 107 passes, and the inner surface of the hole 116 has a recess 125 through which the projections 115, 117, and 119 of the plunger 107 pass (refer to the lower right view for the reference numeral.

There are two recesses 125 located point symmetrically each other about the central axis of the flange cover 109, and the first protruding portion 115 and the second protruding portion 117 preferably correspond to each recesses 125. One recess 125 may be used, but three or more recesses with respect to the central axis of the flange cover 109 is not preferable because it makes the structure more complex and increases cost. The plurality of recesses 125 allows the plunger 107 to be stably fixed to the flange cover 109.

. In order to stabilize the position of the plunger 107 with the flange cover 109, it is preferable to have a plurality of convex portions on the outer surface of the plunger 107 in point symmetry with respect to the central axis of the flange cover 109. The height of the convex portions should be slightly smaller than the diameter of the hole 116. As shown in the embodiment, if the two convex portions are 90° offset from the two projections 115, 117, 119 with respect to the central axis of the flange cover 109, the plunger 107 can be further stabilized.

First, the case at drawing drug solution into the syringe is explained. As shown in the lower right view in Figure 4, the plunger 107 is fully pushed into the syringe 111 and the flange cover 109 is located on the A-A line. The cross-section of the second protruding portion 117 of the plunger 107 is aligned with the recess 125 of the flange cover 109. In other words, the second projection 117 can pass through the flange cover 109.

As shown in the right middle view of Figure 4, as the plunger 107 is pulled out, the flange cover 109 comes onto the B-B line. Up to this point, a fixed amount of drug solution is drawn into the syringe 111. At this point, the cross-section of the continuous protruding portion 119 of the plunger 107 is aligned with the recess 125 of the flange cover 109. In other words, the continuous protruding portion 119 can pass through the flange cover 109.

From this point, when the plunger 107 is further pulled out, the flange cover 109 contacts the inclined surface 121 at the rear end of the first protruding portion 115, and the plunger 107 automatically rotates in the direction indicated by the arrow in the middle right view of Fig. 4. Since the plunger 107 automatically rotates, it is recognized not only visually but also by touch feeling that the plunger 107 is in the metering position. Therefore, further drawing from this position can be used as pre-drawing for air release.

As the plunger 107 rotates from the starting position, the cross-section of the first protruding portion 115 is aligned with the recess 125 of the flange cover 109. In other words, the first protruding portion 115 can pass through the flange cover 109. As shown in the upper right view of Fig. 4, the pre-drawing for air release is completed when the flange cover 109 comes onto the D-D line.

As shown in Figure 5, when the plunger 107 during drug drawing is pulled straight from the position that the second protruding portion 117 is located in the recess 125, the second protruding portion 117, the continuous protruding portion 119, and the first protruding portion 115 pass through the recess 125 in that order. When the inclined surface 121 of the rear end of the first protruding portion 115 passes through the recess 125, the plunger 107 rotates along the inclined surface 121.

Next, air release is explained. As shown in the upper left view of Fig. 4, the flange cover 109 is located on the D-D line of the plunger 107 when the drug solution is drawn. At this time, in the plunger 107, the cross-section of the first protruding portion 115 is aligned with the recess 125. In other words, the first protruding portion 115 passes through the flange cover 109.

As shown in the middle left view of Fig. 4, drug solution is released until the flange cover 109 comes onto the C-C line of the plunger 107, thereby performing air release. At this point, the cross-section of the second protruding portion 117 is partially aligned with the recess 125 of the flange cover 109, but not completely aligned with the recess 125. Therefore, the front end of the second protruding portion 117 of the plunger contacts the flange cover 109.

. Finally, metered dosing is explained. The operator rotates the plunger in the direction of the arrow as shown in the middle left view of Fig. 4, thereby aligning the second protruding portion 117 with the recess 125 of the flange cover 109. This rotational action makes the operator aware that a fixed amount of drug solution is to be dosed from this point. After that, as shown in the lower left figure in Figure 4, the second protruding portion 117 passes through the recess 125 of the flange cover 109, and the plunger 107 is fully pushed into the syringe 111, and then the flange cover 109 comes onto the A-A line of the plunger 107.

. As shown in Figure 5, when the plunger 107 is pushed forward from the position that the first protruding portion 115 is located in the recess 125 for air release, the first protruding portion 115 and the continuous protruding portion 119 pass through the recess in that order, and the front end of the second protruding portion 117 contacts the flange cover 109. Then, the operator rotates the plunger 107 and pushes the plunger 107 further forward, thereby dosing a fixed amount of drug solution.

In this way, it is possible to provide a metered dosing component and a syringe using this metered dosing component, which enables metered dosing other than visually and eliminates incorrect dosing. The present invention also provides a metered dosing component and a syringe using the same to eliminate the waste of drug solution more than required for air release by confirming a metered dosing start position of the plunger. Further, the present invention provides a metered dosing component and an injection device using the same at a low cost by changing the plunger of a general-purpose syringe and adding a flange cover.

### (Metered dosing component according to Second embodiment)

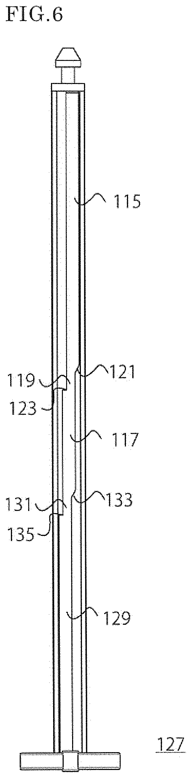

Fig. 6 is a plan view of a plunger according to a second embodiment of the present invention. A second embodiment of the present invention will be described with referring to Fig.6.

. The metered dosing component 112 according to the second embodiment of the invention has a plunger 127, a flange cover 109, and a gasket 113. As shown in Figure 6, the plunger 127 according to the second embodiment has a third protruding portion 129 which extends axially to the rear end side of the second protruding portion 117 of the plunger 127 and is located at a different position from the second protruding portion 117 in the circumferential direction, and a second continuous protruding portion 131 which connects a rear end of the second protruding portion 117 and a front end portion of the third protruding portion 129 in a continuous manner. The plunger according to the second embodiment differs from the plunger according to the first embodiment in this point.

. Therefore, similar to the protruding portions of the plunger according to the first embodiment, when a flange cover 109 is attached to a flange portion 114 of the syringe and the plunger 127 is attached to the syringe and the plunger 127 is pulled backward from the state in which the third protruding portion 129 is located in the recess 125, the third protruding portion 129, the second continuous protruding portion 131 and the second protruding portion 117 pass through the recess 125. When the rear end of the second protruding portion 117 passes through the recess 125 in this order, and when the rear end of the second protruding portion 117 pass through the recess 125, the plunger 127 rotates with respect to the flange cover 109.

This is because the rear end of the second protruding portion 117 has a surface 133 inclined with respect to the axial direction. Similar to the inclined surface 121 according to the first embodiment, the inclined surface 131 is connected to the second continuous protruding portion 131 like a steep ridgeline in the axial and radial direction. The second continuous protruding portion 131 is circumferentially shorter than the second protruding portion 117 and the third protruding portion 129, and is formed to connect the second protruding portion 117 and the third protruding portion 131 at a circumferentially overlapping portion. The second continuous protruding portion 131 preferably extends parallel to the axial direction.

. The front end of the third projection 129 has a surface 135 that is perpendicular to the axial direction. This surface contacts the flange cover 109 and serves as a stopper. Therefore, it is preferably precipitous in the axial and radial direction.

. When the plunger 127 according to the second embodiment is pushed forward from the state in which the third protruding portion 129 is located in the recess 125, the third protruding portion 129 and the second continuous protruding portion 131 pass through the recess 125 in that order, and the front end of the third protruding portion 129 contacts the flange cover 109.

. The metered dosing component 112 allows multiple metering. For example, 0.3 mL is metered by the second continuous projection 131 and another 0.3 mL is metered by the continuous projection 119. Therefore, 0.6 mL can be metered divided in two parts. With one metering, two dosing can be made. Although two times metering is shown in this embodiment, more times metering is achieved, for example, by adding a fourth protruding portion, a fifth protruding portion etc., and connecting them with a third continuous protruding portion, a fourth continuous protruding portion etc., it is possible to meter three times, four times etc.

### (Gasket according to Embodiment)

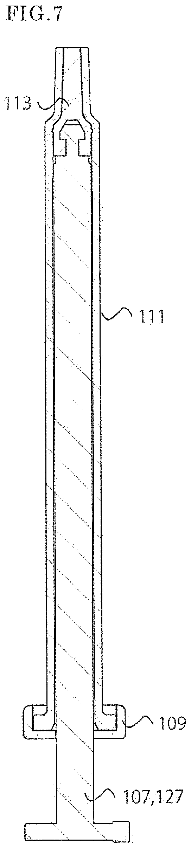

Fig.7 is a cross-sectional view of the injection device pushing the plunger according to the embodiment of the present invention. A gasket according to this embodiment will be described with referring to Fig.7,

. As shown in Figure 7, a shape of an outer surface of the gasket 113 attached to the plunger of this embodiment matches a shape of an inner surface of an end of the syringe 111. Accordingly, a dead space is reduced and very small amounts of drug solution, such as 0.3 mL, are not wasted.

. A metered dosing component 112 has an advantage when dosing 0.1mL, 0.3mL, 0.5mL,...,2mL etc. of drug solution. It has the effect of metering and dosing drug solution with reducing a dead space.

. The present invention can provide a metered dosing component and a syringe using this metered dosing component, which enables metering other than visually and eliminates incorrect dosage. The present invention also provides a metered dosing component and a syringe using the same, to eliminate the waste of drug solution more than required for air release by confirming a metered dosing start position of the plunger. Further, the present invention provides a metered dosing component and an injection device using the same at a low cost by changing the plunger of a general-purpose syringe and adding a flange cover.

. Furthermore, the present invention can provide multiple metered dosing. In addition, the present invention allows metering of even a small amount of drug solution and reduces a dead space.

The above description of embodiments is in all respects illustrative and not restrictive. Variations and modifications are possible from time to time for those skilled in the art. The scope of the invention is indicated by the claims, not by the embodiments described above. Furthermore, the scope of the invention includes changes from the embodiments within the scope of the claims and within the scope of equivalents.

### Industrial applicability.

. Using the metered dosing component provided by the present invention, it is possible to provide a metered dosing component and a syringe using this metered dosing component which eliminate incorrect dosage after puncture because the metering can be completed other than visually before treatment begins.

### Explanation of Reference numeral

- 101: Injection device
- 103: Arrow direction at front end
- 105: Arrow direction at rear end
- 107: Plunger
- 109: Flange cover
- 111: Syringe
- 112: Metered dosing component
- 113: Gasket
- 114: Flange portion
- 115: First protruding portion
- 116: Hole
- 117: Second protruding portion
- 119: Continuous protruding portion
- 121: Inclined surface
- 123: Approximately perpendicular surface
- 125: Recess
- 127: Plunger
- 129: Third protruding portion
- 131: Second continuous projection
- 133: Inclined surface
- 135: Approximately perpendicular surface

## Claims

1. A metered dosing component comprising;
a plunger having a protruding portion on an outer surface thereof,
a flange cover to be placed over a flange portion of a syringe, having a hole through which the plunger passes, and a recess provided in an inner surface of the hole through which the protruding portion passes, wherein
the protruding portion has a first protruding portion extending axially to a front end side of the plunger, a second protruding portion extending axially to a rear end side of the plunger and located at a different position from the first protruding portion in the circumferential direction, and a continuous protruding portion connecting a rear end of the first protruding portion and a front end of the second protruding portion in a continuous manner,
when the flange cover is attached to the flange portion of the syringe and the plunger is inserted into the syringe, and the plunger is pulled backward from a position where the second protruding portion is located in the recess, the second protruding portion, the continuous protruding portion, and the first protruding portion pass through the recess in that order, and when the rear end of the first protruding portion passes through the recess, the plunger rotates with respect to the flange cover.

2. The metered dosing component according to claim 1, wherein
the rear end of the first protruding portion has a surface inclined with respect to the axial direction, and the front end of the second protruding portion has a surface approximately perpendicular to the axial direction,
when the plunger is pushed forward from a state in which the first protruding portion is located in the recess, the first protruding portion and the continuous protruding portion pass through the recess in that order, and the front end of the second protruding portion contacts the flange cover.

3. The metered dosing component according to claim 2, wherein
in order to draw drug solution into the syringe, the plunger inserted deep into the syringe is pulled until the second protruding portion passes through the flange cover, the continuous protruding portion passes through the flange cover while the plunger rotates, and the first protruding portion passes through the flange cover,
in order to perform air release, the plunger is pushed until the front end of the second protruding portion contacts the flange cover, and
in order to perform metered dosing, the plunger is rotated to align the second protruding portion with the recess and then the plunger is pushed in.

4. The metered dosing component according to any one of claims 1 to 3, wherein
two of the recesses are located point symmetrically about the central axis of the flange cover, and
the first protruding portion and the second protruding portion exist in correspondence with the respective recesses.

5. The metered dosing component according to any one of claims 1 to 4, wherein
further, the protruding portion has a third protruding portion extending axially to a rear end of the second protruding portion of the plunger and located at a different position from the second protruding portion in the circumferential direction, and a second continuous protruding portion connecting a rear end of the second protruding portion and a front end of the third protruding portion in a continuous manner,
when the flange cover is attached to the flange portion of the syringe and the plunger is inserted into the syringe and the plunger is pulled backward from a state in which the third protruding portion is located in the recess, the third protruding portion, the second continuous protruding portion, and the second protruding portion pass through the recess in that order,
when the rear end of the second protruding portion passes through the recess, the plunger rotates with respect to the flange cover.

6. The metered dosing component according to any one of claim 5, wherein
the rear end of the second protruding portion has a surface inclined with respect to the axial direction and a front end of the third protruding portion has a surface approximately perpendicular to the axial direction,
when the plunger is pushed forward from a state in which the third protruding portion is located in the recess, the third protruding portion and the second continuous protruding portion pass through the recess in that order, and the front end of the third protruding portion contacts the flange cover.

7. The metered dosing component according to any one of claims 1 to 6, wherein
a shape of an outer surface of a gasket attached to the plunger matches a shape of an inner surface of an end of the syringe.

8. The metered dosing component according to any one of claims 1 to 7, wherein
the metered dosing component is used for dosing 0.1 mL to 2 mL of drug solution.

9. An injection device comprising;
a syringe,
the metered dosing component according to any one of claims 1 to 8, attached to the syringe.
